(19) Europäisches Patentamt
European Patent Office
Office européen des brevets

(11) Veröffentlichungsnummer: **0 236 951**
**A2**

# (12) EUROPÄISCHE PATENTANMELDUNG

(21) Anmeldenummer: **87103139.9**

(22) Anmeldetag: **05.03.87**

(51) Int.Cl.$^3$: **A 61 K 31/415**

(30) Priorität: **06.03.86 DE 3607381**

(43) Veröffentlichungstag der Anmeldung:
**16.09.87 Patentblatt 87/38**

(84) Benannte Vertragsstaaten:
**AT BE CH DE ES FR GB GR IT LI LU NL SE**

(71) Anmelder: **Zimmer, Pascal**
**Lehrertalweg 112**
**D-7900 Ulm(DE)**

(71) Anmelder: **Lehr, Hans Anton**
**Werastrasse 15**
**D-7900 Ulm(DE)**

(72) Erfinder: **Zimmer, Pascal**
**Lehrertalweg 112**
**D-7900 Ulm(DE)**

(72) Erfinder: **Lehr, Hans Anton**
**Werastrasse 15**
**D-7900 Ulm(DE)**

(74) Vertreter: **Kinzebach, Werner, Dr.**
**Patentanwälte Reitstötter, Kinzebach & Partner**
**Sternwartstrasse 4 Postfach 86 06 49**
**D-8000 München 86(DE)**

## (54) Verwendung von Diphenylhydantoin und seinen Derivaten zur Herstellung eines pharmazeutischen Mittels zur Behandlung von Immunerkrankungen.

(57) Die Erfindung betrifft die Verwendung von Diphenylhydantoin und seinen Derivaten zur Herstellung von pharmazeutischen Mitteln zur Behandlung von Immunerkrankungen, insbesondere zur Behandlung von Immunerkrankungen, die durch T-Lymphozyten mediiert werden.

Die erfindungsgemäß zur Anwendung kommenden Verbindungen üben einen spezifischen Maskierungseffekt auf bestimmte Rezeptoren der Lymphozyten aus und sind so in der Lage, einen Angriff der Viren auf die Lymphozyten zu verhindern.

EP 0 236 951 A2

Die Erfindung betrifft die Verwendung von Diphenylhydantoin und seinen Derivaten zur Herstellung eines pharmazeutischen Mittels zur Behandlung von Immunerkrankungen.

Diphenylhydantoin (DPH) wird seit 1938 als Anticonvulsivum eingesetzt. Die systemischen Wirkungen von DPH und dessen Effekt auf das zentrale Nervensystem sind wohl bekannt. Darüber hinaus besitzt DPH eine antiarrythmische Wirkung.

Es wurde nun überraschenderweise gefunden, daß DPH einen spezifischen Einfluß auf die T-Lymphozyten ausübt und deshalb zur Behandlung von Immunerkrankungen geeignet ist.

Die Erfindung betrifft deshalb die Verwendung von Diphenylhydantoin und seinen Derivaten der allgemeinen Formel (I)

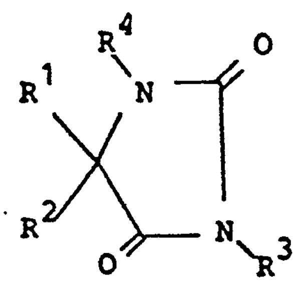

worin

$R^1$ und $R^2$, die gleich oder verschieden sein können, ein Wasserstoffatom, einen Phenylrest, der durch ein Halogenatom, eine Hydroxy-, $C_1$-$C_4$-Alkoxy- oder $C_1$-$C_4$-Alkylgruppe substituiert sein kann, eine $C_1$-$C_4$-Alkylgruppe, die durch ein Halogenatom, eine Hydroxy- oder $C_1$-$C_4$-Alkoxygruppe substituiert sein kann, eine $C_5$-$C_6$-Cycloalkylgruppe oder Thiophengruppe bedeuten und $R^3$ und $R^4$, die gleich oder verschieden sein können,

ein Wasserstoffatom, eine $C_1$-$C_4$-Alkoxy-, $C_1$-$C_4$-Alkyl- oder Phenylgruppe bedeuten, sowie deren pharmazeutisch verträglichen Salze zur Herstellung eines pharmazeutischen Mittels zur Behandlung von Immunerkrankungen.

Vorzugsweise bedeuten $R^1$ und $R^2$ einen gegebenenfalls substituierten Phenyl- oder $C_1$-$C_4$-Alkylrest, z.B. p-Hydroxyphenyl, p-Tolyl, Methyl, Ethyl, n- und i-Propyl, n-Butyl oder t-Butyl.

$R^3$ und $R^4$ bedeuten vorzugsweise ein Wasserstoffatom oder eine $C_1$-$C_4$-Alkylgruppe.

Besonders bevorzugt ist zur Behandlung der Immunerkrankungen Diphenylhydantoin der Formel

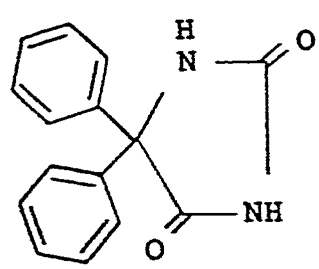

und die Verbindungen der Formeln:

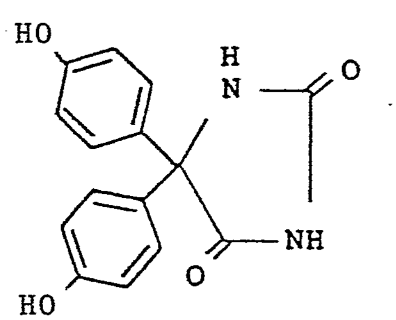

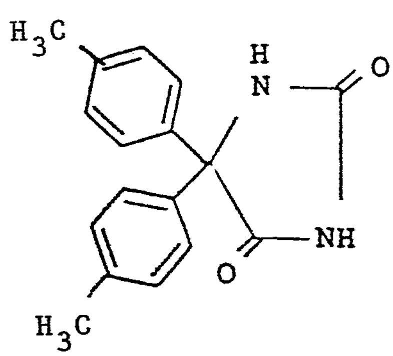

Wie erwähnt, dienen die erfindungsgemäß zur Anwendung kommenden Verbindungen zur Behandlung von Immunerkrankungen, insbesondere zur Behandlung von durch T-Lymphozyten mediierte Immunerkrankungen. Ganz besonders sind die erfindungsgemäßen Verbindungen zur Behandlung von AIDS (Acquired Immun-Deficiency Syndrome) brauchbar.

Anhand von Versuchen, die unten näher erläutert werden, hat sich gezeigt, daß die erfindungsgemäß zur Anwendung kommenden Verbindungen an die Membran lymphoretikulärer Zellen binden und einen spezifischen Maskierungseffekt auf die Rezeptoren von Viren des Typs HTLV-III/LAV ausüben. Dabei ist wesentlich, daß die anderen Funktionen der

Lymphozyten, die sie im Rahmen der Immunantwort haben, nicht beeinträchtigt werden. Die folgenden Versuche bestätigen diesen Sachverhalt:

1. Markierung der Lymphozytenmembran mit immunofluoreszierenden Viren vom Typ HTLV-III/LAV

a) 4 Patienten, die mindestens 10 Tage mit therapeutischen Dosen an Diphenylhydantoin (3 x 100 mg Epanutin p.o./Tag) behandelt worden sind, wurden jeweils 15 ml Blut abgenommen, das nach Versetzen mit Natriumcitrat (2 ml/15 ml Blut) 48 Stunden aufbewahrt wurde.

Die Lymphozyten wurden anhand der bekannten Ficoll/Hypaque-Schichttechnik isoliert. 1 Mio. lebensfähiger Lymphozyten wurden anschließend 1 h bei 4°C zusammen mit 50 ml immunofluoreszierendem HTLV-III/LAV, entsprechend 8000 ng des Virus, inkubiert. Nach zweimaligem 10-minütigem Waschen in Phosphatpufferlösung wurden die Zellen mit einer Mischung aus Phosphatpufferlösung/Glycerin in gleichen Konzentrationen verdünnt. Die Immunofluoreszierenzmarkierung wurde anschließend mit einem Immunofluoreszenzmikroskop bestimmt.

b) In ähnlicher Weise wurde die Markierung der Lymphozytenmembran mit HTLV-III/LAV vorgenommen, indem man jeweils 15 ml heparinisiertes Blut von 4 weiteren Patienten wie unter a) erläutert behandelte. Das Blut wurde vorher mit Heparin stabilisiert und 5 h aufbewahrt.

c) Aus Blut von Patienten, welche vorher nicht mit Diphenylhydantoin behandelt wurden, wurden, wie oben beschrieben, die T-Lymphozyten isoliert. Auf diese T-Lymphozyten ließ man Diphenylhydantoin in Konzentrationen von 2 µg/ml und 20 µg/ml in RPMI 48 h einwirken. Anschließend wurden die Lymphozyten isoliert und mit immunofluoreszierendem HTLV-III/LAV wie oben beschrieben inkubiert. Danach erfolgte die Bestimmung der Immunofluoreszenzmarkierung mit Hilfe eines Immunofluoreszenzmikroskops.

Als Kontrolle dienten Lymphozyten, die aus Blut von Patienten isoliert wurden, welche vorher nicht mit Diphenylhydantoin behandelt wurden. Diese Lymphozyten wurden ohne mit Diphenylhydantoin behandelt zu werden, wie oben beschrieben mit immunofluoreszierendem HTLV-III/LAV inkubiert und ihre Immunofluoreszenzmarkierung bewertet.

Die Ergebnisse sind in den nachfolgenden Tabellen zusammengestellt:

Tabelle I

Ergebnisse des Tests 1a (Stabilisierung des Bluts mit Natriumcitrat)

| Patient[1] Nr. | Markierung der Membran mit immunofluoresz. HTLV-III/LAV % | diffuse Markierung % |
|---|---|---|
| 1 | - | 2,2 |
| 2 | - | 2,3 |
| 3 | - | 1,0 |
| 4 | - | 1,8 |
| Kontrolle | 8,2 | - |

Tabelle II

Ergebnisse des Tests 1b (Stabilisierung des Bluts mit Heparin)

| Patient Nr. | Markierung der Membran mit immunolfuoresz. HTLV-III/LAV % | diffuse Markierung % |
|---|---|---|
| 5 | - | 2,4 |
| 6 | - | 2,6 |
| 7 | - | 1,3 |
| 8 | - | 3,0 |
| Kontrolle | 8,2 | --- |

Tabelle III

Ergebnisse des Tests 1c

| Behandlung der Lymphozyten mit | Markierung der Membran mit immunofluoresz. HTLV-III/LAV % | diffuse Markierung % |
|---|---|---|
| 2 µg/ml DPH | 8,6 | 4 |
| 20 µg/ml DPH | 1,5 | 2,5 |
| Kontrolle | 8,9 | 5 |

2. Lymphozyten, die mit DPH behandelt worden waren, wurden anschließend mit monoklonalen Antikörpern (OKT 4,4 OKT 8, OKT 3, OKT 11) markiert und das Markierungsmuster bewertet. Das Markierungsmuster unterschied sich nicht von dem Muster, das unbehandelte T-Lymphozyten zeigen.

Aus den Tabellen I und II ist ersichtlich, daß bei Lymphozyten, die von den mit Diphenylhydantoin behandelten Patienten stammen bzw. die in vitro mit

Diphenylhydantoin behandelt wurden, keine Immunofluoreszenzmarkierung stattfindet. Die so behandelten T-Lymphozyten zeigen lediglich eine geringe diffuse Markierung.

Wie erwähnt, ist dieser Befund so zu erklären, daß Diphenylhydantoin an die Membran der Lymphocyten bindet und die Rezeptoren für HTLV-III/LAV markiert, so daß die Viren nicht mehr in der Lage sind, sich an die Zellmembran anzulagern oder sie zu durchdringen.

Dagegen zeigen die Daten für die unbehandelten Kontrollproben, daß die Membran der T-Lymphozyten von immunofluoreszierendem HTLV-III/LAV markiert und somit angegriffen wird.

Der Test 2 belegt, daß die erfindungsgemäßen zur Anwendung kommenden Diphenylhydantoin-Derivate die Rezeptoren für Viren vom Typ HTLV-III/LAV spezifischen maskieren, d.h. ein Angriff von HTLV-III/LAV kann nicht erfolgen, während die anderen Funktionen der Lymphozyten im Rahmen der Immunantwort aufrechterhalten bleiben.

Darüber hinaus führt die Verabreichung der erfindungsgemäß zur Anwendung kommenden Verbindungen dazu, daß die im Knochenmark neugebildeten Lymphozyten noch vor der Differenzierung in B- und T-Lymphozyten bzw. in spezifische T-Zellsubpopulationen, insbesondere T-Helferzellen, in gleicher Weise beeinflußt und damit gegen einen Virusangriff geschützt werden. Dies führt dazu, daß im Organismus eine wachsende Population geschützter, gesunder T-Lymphozyten vorliegt, die im Laufe der Zeit die infizierten Lymphozyten ersetzen.

Die erfindungsgemäß zur Anwendung kommenden Diphenylhydantoin-Derivate eignen sich deshalb zur prophylaktischen, palliativen und kurativen Behandlung von Immunerkrankungen, insbesondere zur Behandlung von durch T-Lymphozyten mediierten Immunerkrankungen. Ganz besonders sind diese Verbindungen zur Behandlung von AIDS brauchbar.

Die Verbindungen können alleine oder auch in Kombination mit weiteren, geeigneten Wirkstoffen zur Anwendung kommen. Außerdem bieten sich bestimmte Begleittherapien, wie Einpflanzung von Knochenmark oder Thymus, Gaben von HLA-identischen Lymphozyten und Gaben von Thymushormon oder -extrakt an.

Die Diphenylhydantoin-Derivate können oral oder parenteral vorzugsweise in Form eines Salzes, beispielsweise des Natriumsalzes, verabreicht werden. Bei oraler Verabreichung werden diese Verbindungen in üblichen Darreichungsformen, z.B. als Tablette, Kapsel und dergl., zusammen mit üblichen Zusatz- und Hilfsstoffen konfektioniert. Für Injektionspräparate werden diese Verbindungen in der Regel als Natriumsalz eingesetzt und in einem geeigneten Lösungsmittel oder einem Lösungsmittelgemisch gelöst, z.B. Wasser in Mischung mit Glykolen oder Glycerin.

Die Dosierung der Diphenylhydantoin-Derivate erfolgt je nach Art und Schwere der Erkrankung. Ein Bereich von 10 bis 1500 mg/pro Tag, vorzugsweise 100 bis 1000 mg/pro Tag ist als zweckmäßig anzusehen. In der Regel ist eine Dosierung von 300 mg/pro Tag geeignet.

Formulierungsbeispiele:

1. Kapsel

100 mg Diphenylhydantoin werden in eine Weichgelatinekapsel gefüllt und können dann so verabreicht werden.

2. Infusionslösung

12 % Diphenylhydantoin-Na-Salz
16 % Tetraglykol
11 % Trispuffer
Injektionswasser ad 100 %

3. Injektionspräparat

Diphenylhydantoin-Na-Salz 250 mg
Tetraglykollösung ad 5 ml

---

## Patentansprüche

1. Verwendung von Diphenylhydantoin und seinen Derivaten der allgemeinen Formel (I):

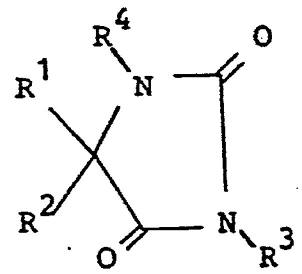

worin

$R^1$ und $R^2$, die gleich oder verschieden sein können, ein Wasserstoffatom, einen Phenylrest, der durch ein Halogenatom, eine Hydroxy-, $C_1$-$C_4$-Alkoxy- oder $C_1$-$C_4$-Alkylgruppe substituiert sein kann, eine $C_1$-$C_4$-Alkylgruppe, die durch ein Halogenatom, eine Hydroxy- oder $C_1$-$C_4$-Alkoxygruppe substituiert sein kann, eine $C_5$-$C_6$-Cycloalkylgruppe oder Thiophengruppe bedeuten und $R^3$ und $R^4$, die gleich oder verschieden sein können, ein Wasserstoffatom, eine $C_1$-$C_4$-Alkoxy-, $C_1$-$C_4$-Alkyl- oder Phenylgruppe bedeuten,

sowie deren pharmazeutisch verträglichen Salze

zur Herstellung eines pharmazeutischen Mittels zur Behandlung von Immunerkrankungen.

2. Verwendung nach Anspruch 1 von Diphenylhydantoin der Formel:

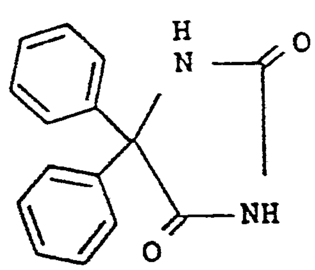

und der pharmazeutisch verträglichen Salze davon.

3. Verwendung nach Anspruch 1 der Verbindung der Formel:

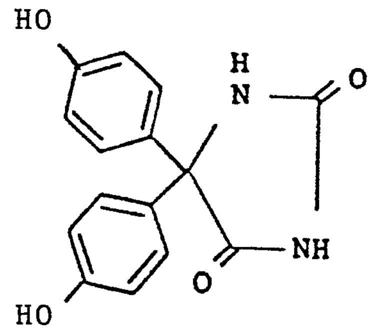

und der pharmazeutisch verträglichen Salze davon.

4. Verwendung nach Anspruch 1 der Verbindung der Formel:

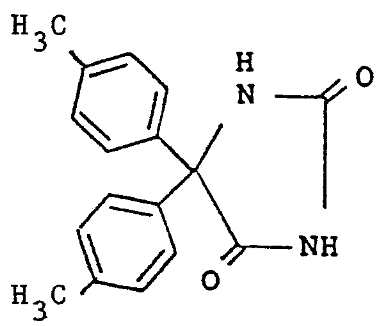

und der pharmazeutisch verträglichen Salze davon.

5. Verwendung nach Anspruch 1 zur Behandlung von Immunerkrankungen, welche durch T-Lymphozyten mediiert sind.

6. Verwendung nach Anspruch 5 zur Behandlung von AIDS.